**Europäisches Patentamt**

**European Patent Office**

**Office européen des brevets**

(11) Publication number : **0 370 673 B1**

# (12) EUROPEAN PATENT SPECIFICATION

(45) Date of publication of patent specification :
**28.04.93 Bulletin 93/17**

(51) Int. CI.⁵ : **A61K 35/84**

(21) Application number : **89311733.3**

(22) Date of filing : **13.11.89**

(54) **Therapeutic agent for aids.**

(30) Priority : **14.11.88 JP 287316/88**

(43) Date of publication of application :
**30.05.90 Bulletin 90/22**

(45) Publication of the grant of the patent :
**28.04.93 Bulletin 93/17**

(84) Designated Contracting States :
**DE FR GB**

(56) References cited :
**EP-A- 0 088 151**
**EP-A- 0 154 066**
**EP-A- 0 292 601**
**EP-A- 0 295 962**

(73) Proprietor : **NODA SHOKUKIN KOGYO CO., LTD.**
**121, Shimizu**
**Noda-shi Chiba-ken 278 (JP)**

(72) Inventor : **Iizuka, Chiyokichi, c/o NODA SHOKUKIN KOGYO CO LTD**
**121 Shimizu, Noda-shi**
**Chiba-ken, Japan 278 (JP)**
Inventor : **Iizuka, Hiroshi, c/o NODA SHOKUKIN KOGYO CO LTD**
**121 Shimizu, Noda-shi**
**Chiba-ken, Japan 278 (JP)**
Inventor : **Ohashi, Yasuhiro, c/o NODA SHOKUKIN KOGYO CO LTD**
**121 Shimizu, Noda-shi**
**Chiba-ken, Japan 278 (JP)**

(74) Representative : **Holmes, Michael John Frank B. Dehn & Co. European Patent Attorneys Imperial House 15-19 Kingsway London, WC2B 6UZ, (GB)**

## Description

The present invention relates to a therapeutic agent for acquired immune deficiency syndrome (hereinafter referred to as "AIDS"). In particular the invention relates to a therapeutic agent produced by cultures of basidiomycete fungi and to a process for preparing the same.

Human immunodeficiency viruses (hereinafter referred to as "HIV") are human RNA viruses belonging to the Retrovirus family, sub-family Lentivirus. Such viruses are known to have an affinity for $T_4$ positive T cells, resulting in death of the infected cells. Subjects who have been infected with these viruses are thus brought into immunological deficiency, leading to the acquired immune deficiency syndrome (AIDS), and secondary conditions such as Karposi's sarcoma and opportunistic infectious diseases. According to Statistics obtained by the Centres for Disease Control (CDC) in the U.S.A., the number of cases in the U.S.A. reached 11,871 in July 12, 1985, and of these 5,917 cases have died. Since then the number of patients has been increasing exponentially. In Japan both patients exhibiting full-blown AIDS and antibody carriers have also been found, and urgent countermeasures against the disease are being sought.

Retroviruses have a genome comprising two molecules of RNA and a peripheral envelope the glycoprotein of which is responsible for the antigenicity of the viruses. Retroviruses are characterized by exhibiting the activity of reverse transcription. More specifically, the virus is first adsorbed on the surface of a cell, where the envelope is removed, and then invades the cell. Uncoating of the virus takes place within the cell by the action of reverse transcriptase serving as a catalyst, and reverse transcription is started from the RNA genome. A part of the double helical DNA produced as a result of the reverse transcription is incorporated in the host cell DNA. This DNA is transcribed and the RNA thus produced functions both or genomic viral RNA and as viral mRNA. Once protein synthesis has occured, the RNA molecules cover themselves with a cell membrane at the surface of the cell, followed by budding and rupture of the cell. The, viruses are thus multiplied.

The present inventors have for many years investigated the mycelia of fungi belonging to the Basidiomycete genus, such as Lentinus edodes (shiitake mushroom). As a result, they have found that a substance extracted from a culture product of the mycelia of Basidiomycetes such as Lintinus edodes, has an immunopotentiation action (see Japanese Patent Publication No. 23392/1978 and EP-A-0154066), is effective as an antiviral agent (Japanese Patent Publication No. 36009/1987), and effective against chronic hepatitis type B (Japanese Unexamined Patent Publication No. 270532/1987). An anti-cancer effect has also been suggested (EP-A-0088151).

It was not however clear from such studies whether the substance extracted from a culture product of the mycelia of Basidiomycetes was effective against viruses such as the AIDS virus which replicate by a mechanism as described above and which specifically infect human beings. When taking into account the replication mechanism mentioned above, a possible approach in the treatment of AIDS and other retroviral infections may be to inhibit the activity of the reverse transcriptase in order to suppress the replication of such viruses. In EP-A-0292601 and EP-A-0295962 of Noda Shokukin Kogyo Co., Ltd (of earlier priority date but published after the present priority date) the use of Basidiomycete extracts in treating AIDS is suggested.

It is an aim of the present invention to provide a therapeutic agent for AIDS, that can suppress the replication of human immunodeficiency viruses whereby to normalize immunological mechanisms, and which is also safe to humans and free from any side effects.

The present inventors have carried out intensive studies to achieve the above object. As a result, we have found that a component extracted from a culture product of mycelia of Basidiomycetes can effectively suppress the replication of human immunodeficiency viruses.

In one aspect the present invention therefore provides a therapeutic agent for the treatment of human immunodeficiency virus (HIV) infections comprising as an active component a hot-water-soluble extract of the mycelia of fungi of the genus Basidiomycetes, said active component being soluble at an aqueous ethanol concentration of 37.5% and insoluble at an aqueous ethanol concentration of 50%.

According to a further aspect, the present invention provides a process for the preparation of a therapeutic agent for the treatment of human immunodeficiency virus (HIV) infections, said process comprising the steps of:

(a) culturing mycelia of Basidiomycetes;
(b) causing the said mycelia to undergo autolysis;
(c) extracting the mycelial contents using hot water;
(d) subjecting the resulting extract to alcohol precipitation; and
(e) collecting a fraction which is soluble at an aqueous ethanol concentration of 37.5 % and insoluble at an aqueous ethanol concentration of 50 %.

As will be evident from test Examples hereinafter described the therapeutic agent of the present invention is effective in inhibiting infection of human subjects with human immunodeficiency viruses. Moreover, because the substance is extracted from a 100% natural product, anxieties concerning potential side effects are much reduced when compared to synthetic chemical agents, and the product of the invention is therefore very safe.

The agent is effective when administered both orally and parenterally and therefore is particularly suitable for the treatment of patients infected with human immunodeficiency viruses who need to be treated for the rest of their lives.

It is not entirely clear why the therapeutic agent of the present invention has the superior effect of inhibiting infection by human immunodeficiency viruses but it is thought that it may act to inhibit the adsorption of human immunodeficiency viruses onto host cells and also to inhibit the activity of reverse transcriptase.

According to the process for preparing the therapeutic agent of the present invention, it is also possible to separate the active component from the crude culture extract and optionally to further concentrate or purify it.

The invention will now be described in more detail with reference to the following Figures:

Fig. 1 is a graph showing the inhibitory effect of LEM-HT against infection with human immunodeficiency viruses;

Fig. 2 is a flow chart of the fractionation of LEM-HT following an alcohol precipitation method;

Fig. 3 is a graph showing the inhibitory effect of LEM-HT (E-3) against infection with human immunodeficiency viruses;

Fig. 4 is a graph showing the effect of LEM-HT in enhancing the activity of interleukin-1;

Fig. 5 is a flow chart of the fractionation of LEM-HT;

Fig. 6 is a graph showing the inhibitory effect against infection with HIV, of each fraction obtained as a result of the above fractionation; and

Fig. 7 is a graph to show the inhibitory effect against reverse transcriptase of a neoPPT1 fraction and a EP3 fraction.

The present invention will be described below in greater detail by giving preferred embodiments of the invention.

Basidiomycete fungi which may be used according to in the present invention include for example <u>Lentinus edodes</u>, <u>Coriolus versicolor</u>, <u>Pleurotus ostreatus</u>, <u>Flammulina Veltipes sing</u>, <u>Ganoderma lucidum</u>, and <u>Grifola frondosa</u>. Of these <u>Lentinus edodes</u> is particularly preferred. All these Basidiomycetes are fungi which are known in the art and are readily available.

In the present invention, mycelia of these Basidiomycetes are cultured, and the active component is extracted from the resulting culture product. Any solid or liquid medium capable of supporting growth of Basidiomycetes may be used, but it is preferred to include in the medium a material prepared from true grasses. The material prepared from true grasses includes, for example, bagasse, wheat straw, rice plant straw, corn stems and leaves, rice bran, and wheat bran. Particularly preferred is bagasse. It is presumed that the material prepared from true grasses is degraded by the Basidiomycetes and it may thus effectively form, together with a component directly produced by mycelia of Basidiomycetes, the component effective for the therapy of AIDS. Other nutrient components may be added to the medium, such as sawdust, peptone, yeast, and sugar cane waste syrup.

The mycelia of Basidiomycetes may be cultured, by inoculating into a medium as described above, a mycelial pellet obtained by liquid culture of spores of Basidiomycetes. If a solid medium is used, the inoculated cultures are incubated for 3 to 6 months in a culture chamber air-conditioned to a temperature of about 18 to 25°C and a humidity of about 50 to 90%. Most preferably, the cultures are incubated for 4 to 6 months in a culture chamber air-conditioned to a temperature from 20 to 25°C and a humidity of 60%. Preferably, once the mycelia have spread in the media the cultures may be moved to a temperature treatment chamber and subjected to temperature variation treatment. The temperature variation treatment is caried out, for example, by first heating the cultures at 32 to 34°C for 24 to 48 hours, and then moving the cultures to a low temperature treatment chamber, where a low temperature treatment is caried out at 4 to 8°C at a humidity of 85% for 5 to 7 days. This temperature variation treatment is preferably employed since it results in stabilization of the quality of the products, but it is not an essential step. Thereafter, the cultures are moved to a cultivation chamber and left to stand. As a result, fruit bodies begin to develop, but the culture is stopped at this stage, and the resulting culture product is crushed using a crusher, as hereinafter described. Alternatively, if a liquid medium is used, the mycelia are cultured by aerated culture or shaking culture, at a temperature of from 15 to 30° for about 1 week to about 1 month. The culture is stopped in the state in which the mycelia have spread in the medium.

After the culture has been completed, the mycelia are caused to undergo autolysis catalysed by the enzymes present inside the mycelia, and a metabolite is extracted. In the case of culture on a solid medium, a preferred extraction method comprises first crushing the resulting culture product and then treating the crushed product at 40 to 9°C for about 3 to 6 hours to cause autolysis catalysed by the mycelial enzymes. Most preferably, the crushed product is heated under aeration of about 80°C for 3 to 4 hours to accelerate the enzyme reactions which cause the mycelia to undergo autolysis, and is dried to a water content of about 3 to 5%. Next, hot water at a temperature of 40°C or more is poured on the resulting crushed product to extract the active component. In a most preferred embodiment, about 5 $\ell$ of water is added to 600 g of the crushed product, and the mixture is boiled for about 1 hour with stirring. As a result of this stirring, the active component contained in the metabolite of mycelia and mycelium cell is extracted into the hot water. The suspension thus obtained is filled into a filtration bag made of, for example, flannel cloth, which is pressed to effect filtration, and the resulting filtrate is further filtered through a membrane filter to remove bacteria, thereby obtaining an extract solution in which the active component is contained. Alternatively if a liquid medium is used, the mycelia are optionally crushed, and thereafter heated to 40°C to 60°C to cause the mycelia to undergo autolysis, thereby obtaining a liquid suspension culture product in which the mycelia are dissolved. This culture product may be subjected to the filtration and removal of bacteria in the same manner as described above for the solid medium to obtain an extract solution. The resulting extract solution may also be optionally concentrated by means of a ultrafiltration membrane an evaporator, followed by freeze-drying thereof to give a brown powder.

As an example of a method suitable for purifying the active component from the crude extract solution, the following method can be employed.

Water to the above-described dried powder, preferably in about 10-fold volume, to prepare a suspension with a pH of about 7.2. Ethyl alcohol (which may be replaced by a lower alcohol of different type) is added to the suspension to obtain a precipitate. As will be evident from the Examples set out later, the replication suppressive activity against human immunodeficiency viruses is particularly strongly seen in the fraction which is soluble at an alcohol concentration of 37.5% but becomes insoluble at an alcohol concentration of 50%. Hence, according to the invention the fraction soluble at an alcohol concentration of 37.5% and insoluble at an alcohol concentration of 50% is collected.

The alcohol precipitate thus obtained may be further passed through a chromatography column (chromatocolumn) of various types to separate the active fraction. Suitable chromatocolumns include, for example, a Con A Sepharose column, a lentyl lectin Sepharose column, a phenyl Sepharose Cl-4B column, and a hydroxy apatite column. The alcohol precipitate may be adsorbed onto any of these chromatocolumns and the desired fraction may be eluted using a given eluent and collected. In a preferred column chromatography fractionation step, the alcohol precipitate is passed through a phenyl Sepharose column and the fraction eluted with 75% ethylene glycol is then collected. This fraction shows a more remarkable replication suppressive activity against human immunodeficiency viruses.

The therapeutic agent of the present invention can be administered to human bodies orally or parenterally for example intravenously, subcutaneously or intramuscularly. Oral administration, however, is most suitable taking into account the nature of the therapy.

In the case of the oral administration, there are no particular limitations on dosage, but the agent may preferably be administered in an amount of from 1 g to 9g, and more preferably from 3 g to 6 g, a day per patient. A therapeutic effect on AIDS can thus be exhibited.

The present invention will be described below in further detail by the following Examples.

EXAMPLES

Example 1

(1) Culture of Lentinus edodes mycelia:

A solid medium mixed with 90% of bagasse, 5% of rice bran and 5% of a nutrient source such as wheat bran was sterilized according to a conventional method, and a solid seed fungi (or mycelial pellets obtained by liquid culture) of Lentinus edodes were inoculated therein. Thereafter, the medium was moved to the inside of a culture chamber air-conditioned to a temperature of from 20 to 25°C and a humidity of 60%, and was cultured for 4 to 6 months. After the mycelia had spread in the medium, the culture was moved to a temperature treatment chamber and heated there at 32 to 34°C for 24 to 48 hours. It was then moved to a low temperature treatment chamber, and subjected to a low temperature treatment at 5 to 8°C at a humidity of 85% for 5 to 7 days. Thereafter, the culture was moved to a cultivation chamber and left to stand there. When fruit bodies began to develop, the culture was removed and was then crushed using a crusher.

(2) Extraction of active component from culture product:

The crushed product obtained as described above was heated under aeration at about 80°C for 3 to 4 hours to accelerate the reaction of enzymes and cause the mycelia to undergo autolysis, and also dried to a water content of about 3 to 5%. About 5 $\ell$ of water was added to 600 g of this crushed product, followed by boiling for about 1 hour with stirring. As a result of this stirring, the active component contained in the metabolite of mycelia and mycelial cytoplasm was extracted into the hot water.

The suspension thus obtained was filled in a filtration bag made of flannel cloth, which was pressed to effect filtration, thus obtaining a filtrate. The resulting filtrate was further filtered through a membrane filter to remove bacteria, thereby obtaining an extract solution. This extract solution was concentrated under pressure, followed by freeze-drying to obtain a brown powder. This powder was designated as LEM-HT.

Analysis of components of the above LEM-HT revealed the following: Sugar: 44.0%; protein: 24.6%; others: 31.4%. The sugar was determined according to a phenol/sulfuric acid method, and the protein was determined according to a Lowry method.

Determination of the sugar composition of the polysaccharide constituting the main component of LEM-HT revealed the following: Glucose: 27.5%; galactose: 3.1%; mannose: 6.7%; xylose: 30.9%; arabinose: 30.5%; fucose: 0.7%; rhamnose: 0.6%.

(3) Infection inhibitory effect of LEM-HT in a human immunodeficiency viruses-cultured human T cell system:

i) Test method

HTLV-1 positive MT-4 cells (cultured human T cell system) were infected with HIV with a multiplicity of infection of 0.001 (proportion of 1 virus to 1,000 cells), and the number of cells was adjusted to $2 \times 10^5/m\ell$ in a 10% FCS-added RPMI-1640 culture medium. The resulting cell suspension was pipetted respectively into given wells of a tissue culture plate having 24 holes. On the other hand, LEM-HT was added respectively into the wells of the above plate so as to give a final concentration of 0.1 mg/m$\ell$, 0.25/m$\ell$ or 0.5 mg/m$\ell$, and then maintained at 37°C in 5% $CO_2$-added moist air. The culture medium was exchanged every 3 days, and LEM-HT was added every time it was exchanged. The number of the cells on which HIV antigens appeared was measured every 3 days according to an indirect immunofluorescene method, and then compared with controls in which no LEM-HT was added.

ii) Test results

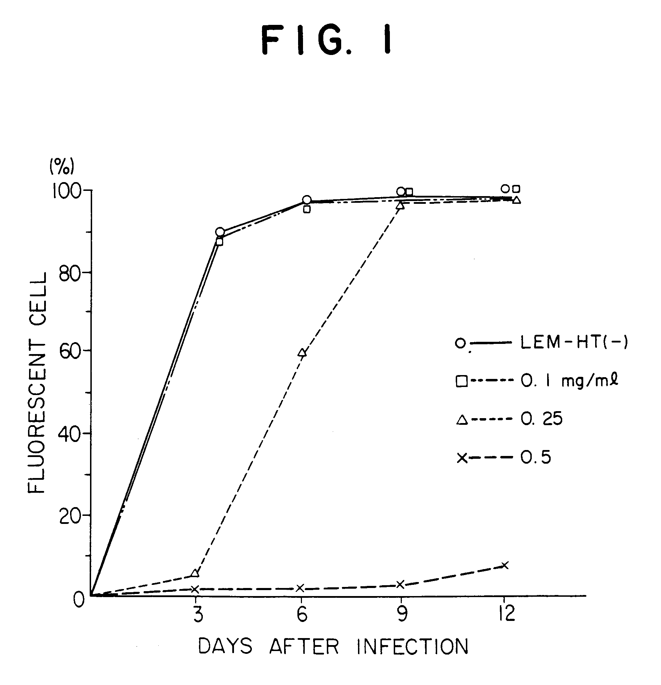

Test results obtained are as shown in Fig. 1. In Fig. 1, the ordinate indicates the proportion (%) of HIV antigen positivecells, and the abscissa the days after infection.

In controls, 90% of cells turned positive for HIV antigens on the 3rd day after culture, and almost all cells were dead on the 9th day after culture. LEM-HT, however, showed an inhibitory effect proportionally depending on its dosage, against the infection with HIV on MT-4 cells. This inhibitory effect showed that infection was almost completely inhibited for 12 days at a concentration of 0.5 mg/m$\ell$.

(4) Purification using alcohol precipitates:

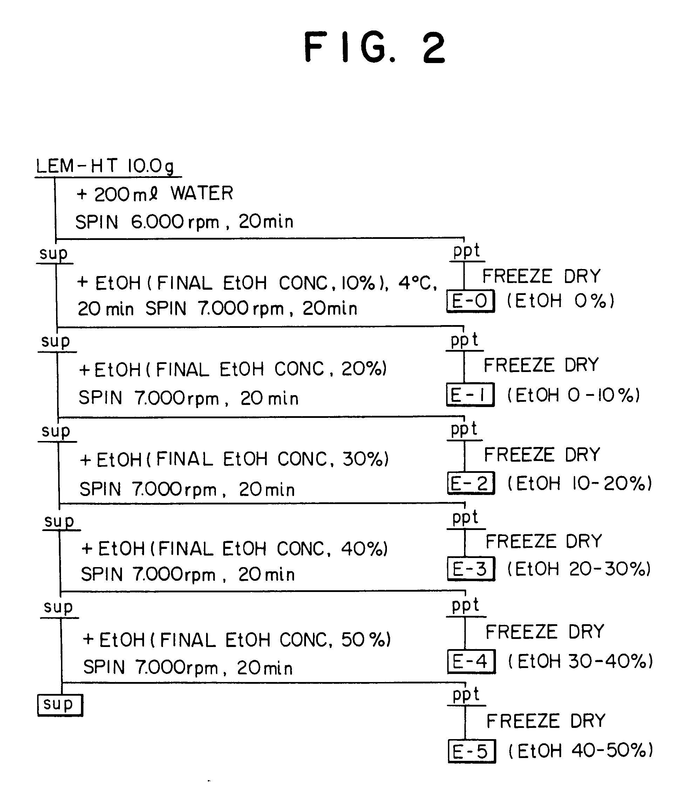

LEM-HT was purified according to the flow chart as shown in Fig. 2.

(i) LEM-HT (10 g) was dissolved in 200 m$\ell$ of water, followed by centrifugal separation at 6,000 rpm for 20 minutes. A precipitate thus obtined with an alcohol concentration of 0% was freeze-dried, and thus was designated as E-0.

(ii) Next, 22.2 m$\ell$ of ethyl alcohol was added to 200 m$\ell$ of the supernatant obtained in the step (i) to give an alcohol concentration of 10%, and then left to stand at 4°C for 20 minutes followed by centrifugal separation at 7,000 rpm for 20 minutes. A precipitate thus obtained with an alcohol concentration ranging from 0 to 10% was freeze-dried, and this was designated as E-1.

(iii) Next, 25 m$\ell$ of ethyl alcohol was added to 200 m$\ell$ of the supernatant obtained in the step (ii) to give an alcohol concentration of 20%, followed by centrifugal separation at 7,000 rpm for 20 minutes. A precipitate thus obtained with an alcohol concentration ranging from 10 to 20% was freeze-dried, and this was designated as E-2.

(iv) Next, 28.6 m$\ell$ of ethyl alcohol was added to 200 m$\ell$ of the supernatant obtained in the step (iii) to give an alcohol concentration of 30%, followed by centrifugal separation at 7,000 rpm for 20 minutes. A precipitate thus obtained with an alcohol concentration ranging from 20 to 30% was freeze-dried, and thus was designated as E-3.

(v) Next 33.3 m$\ell$ of ethyl alcohol was added to 200 m$\ell$ of the supernatant obtained in the step (iv) to give an alcohol concentration of 40%, followed by centrifugal separation at 7,000 rpm for 20 minutes. A precipitate thus obtained with an alcohol concentration ranging from 30 to 40% was freeze-dried, and thus was designated as E-4.

(vi) Next, 40 m$\ell$ of ethyl alcohol was added to 200 m$\ell$ of the supernatant obtained in the step (v) to give an alcohol concentration of 50%, followed by centrifugal separation at 7,000 rpm for 20 minutes. A precipitate thus obtained with an alcohol concentration ranging from 40 to 50% was freeze-dried, and this was designated as E-5.

(5) Tests on human immunodeficiency viruses inhibitory action, using the above E-3 Precipitate fraction:

i) Test method

Once ATH8 cells (HTLV-I positive, human T cells) are infected with human immunodeficiency viruses, viable cells gradually decrease. Then, in a culture medium of ATH8 cells ($2 \times 10^5$ per culture), the above fractioned specimen E-3 was added so as to give a concentration of from 0.1 to 200 $\mu$g/m$\ell$. In the resulting cell mixture, HIV (2,000 virus particles per cell) was inoculated, followed by culture at 37°C for 7 days. Thereafter, the decrease in viable cells wash compared with that of controls which were not infected with HIV.

ii) Test results

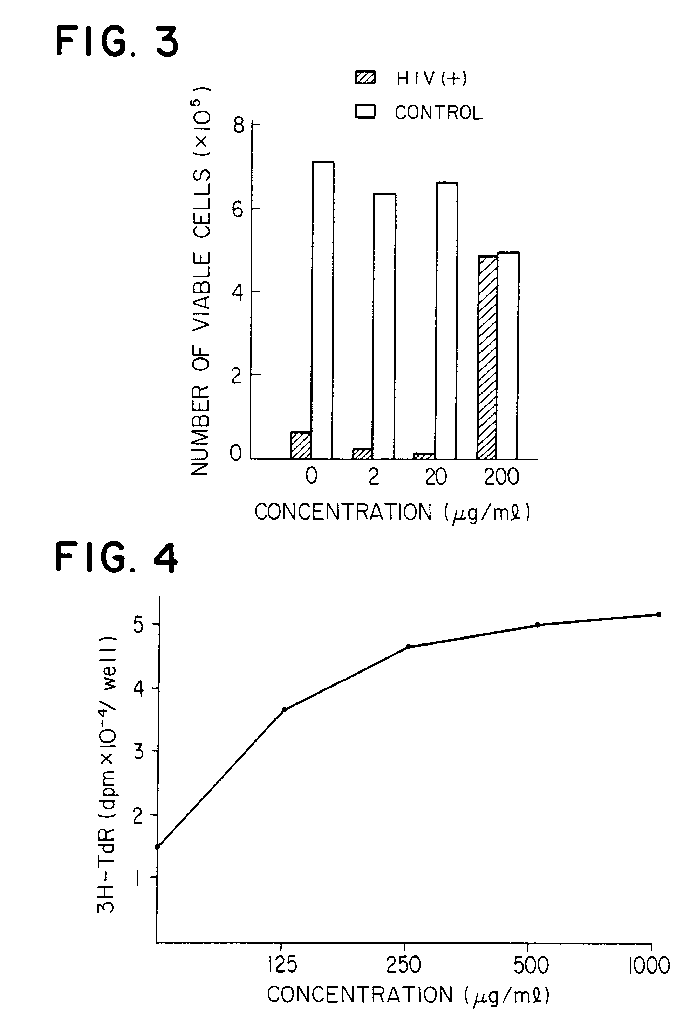

Test results obtained are shown in Fig. 3. As will be evident from Fig. 3, it was possible to substantially inhibit the decrease in viable cells which is due to the infection with HIV, when 200 $\mu$m/m$\ell$ of the present substance (E-3) was added in the target cells. Thus, there was recognized a clear antiviral action.

(6) Tests on immunopotentiational action of LEM-HT

i) Test method

Sterilized liquid paraffin was interperitoneally injected in a guinea pig, and peritoneal exudate macrophages were collected after 4 days. The number thereof was adjusted to $5 \times 10^6$ cells/m$\ell$ in an Eagle MEM medium. In the resulting cell suspension of the microphages, LEM-HT was added so as to give a concentration of from 125 to 1,000 $\mu$g/m$\ell$, and culture was carried out at 37°C for 6 hours. After the culture was completed, the cells were washed twice with the medium to remove LEM-HT, followed by culture in a 10% FCS-added Eagle MEM medium for 42 hours. Interleukin (IL-1) contained in the supernatant produced by the LEM-HT-treated macrophage culture was measured by the thymocyte proliferation assay. More specifically, the supernatant resulting from the LEM-HT-treated macrophage culture was added to the cells obtained from C3H/HeJ mouse thymus, to which 2 $\mu$g/m$\ell$ of phytohemagglutinin was further added, followed by culture for 48 hours. Thereafter, $3,7.10^{-16}.s^{-1}$/ml (1 $\mu$Ci/m$\ell$) of $^3$H-thymidine was added to further continue the culture for 24 hours. Then, the amount of incorporation of $^3$H-thymidine into an acid-insoluble fraction was measured using a liquid scintillation counter.

ii) Test results

As will be evident from Fig. 4, LEM-HT stimulated the macrophages at a concentration of not less than 125 $\mu$g/m$\ell$ and enhanced the activity in the production of IL-1. This indicates that LEM-HT enhances the productive activity of IL-1 as a result of its action on the macrophage cells, and thus increases the ability of lymphocytes in quality to enhance the ability of making antibodies.

(7) Tests on mitogenic effect of LEM-HT on mouse splenic cells:

i) Test method

A spleen was delivered from a rat, and cut to pieces in a physiological saline, followed by filtration on a stainless steel wire net of 80 mesh. Filtrate suspension was subjected to centrifugal separation, and the resulting pellets were suspended in a 0.35% saline solution to make a hemolytic treatment. The resulting suspension was subjected to centrifugal separation to remove the supernatant. Next, the cells sedimented were washed with a Hanks' solution containing kanamycin (60 $\mu$g/m$\ell$). The cells thus washed were dispersed in a TC 199 medium (available from Difco Co.) containing 20% (v/v) of bovine fetal serum (FBS; available from M.A. Bioproduct Co.) and 0.006% (w/v) of kanamycin sulfate, so as to give a cell number of $10^7$ cells/m$\ell$. The resulting

suspension was pipetted into given wells of a well plate, 100 $\mu\ell$ in each, and LEM-HT solutions prepared to have various concentrations were each similarly pipetted, followed by culture in the presence of 5% $CO_2$ at 37°C for 24 hours. [Methyl-$^3$H]thymidine ([$^3$H]-TdR; available from Amersham Japan Co.) was further added by $0.37.10^{-16}.s^{-1}$ (0.1 µCi) per one well, followed by culture for 24 hours. These cells were agglutinated in the form of a sheet on a glass fiber membrane, which were then washed three times with cold physiological saline - cold 5% (w/v) trichloroacetic acid, twice with cold 95% ethyl alcohol, and further once with cold diethyl ether, followed by air-drying. Finally, the radioactivity thereof was measured using a liquid scintillation counter.

ii) Test results

Test results obtained are as shown in the following table. In the table, $\pm\sigma_{n-1}$ is obtained according to the following equation:

$$\pm\sigma_{n-1} = \Sigma(Xi - X)^2/(n - 1)$$

## TABLE

| Mouse No. | Dose of LEM-HT ($\mu$g/culture) | | | | | |
|---|---|---|---|---|---|---|
| | 0.4 | 1 | 2 | 4 | 10 | 20 |
| 1 | 1.39 | 1.33 | 1.51 | 1.08 | 2.46 | 2.25 |
| 2 | 0.98 | 1.00 | 1.18 | 1.51 | 2.13 | 2.01 |
| 3 | 1.01 | 1.20 | 1.39 | 1.46 | 2.02 | 2.10 |
| 4 | 1.46 | 1.37 | 1.68 | 1.82 | 2.35 | 2.49 |
| 5 | 1.16 | 1.37 | 1.38 | 1.63 | 2.23 | 2.00 |
| 6 | 1.27 | 1.56 | 1.38 | 1.75 | 2.41 | 2.38 |
| Average | 1.21 | 1.31 | 1.42 | 1.69 | 2.27 | 2.21 |
| $\pm\sigma_{n-1}$ | 0.20 | 0.19 | 0.17 | 0.20 | 0.17 | 0.20 |

As will be seen from this table, LEM-HT promotes the blastogenesis of mouse spleen cells at a concentration of not less than 0.4 µg/culture, and thus has a clear mitogenic effect.

As demonstrated in the above, LEM-HT was confirmed to promise direct effectiveness in inhibiting infection with human immunodeficiency viruses and suppressing the replication thereof, at the same time to strengthen the immunological competence as a result of its action on living bodies, and to have a therapeutic effect. Namely, in view of the facts that a) LEM-HT suppresses the decrease in T-lyphocytes which is caused by infection with human immunodeficiency viruses, b) it activates macrophages, augments the production of IL-1, and promotes the production of antibodies, and c) it has a mitogenic activity on spleen cells, LEM-HT is considered to be very effective for suppressing the infection with, and replication of, human immunodeficiency viruses.

(8) Tests on acute toxicity of LEM-HT:

Using rats of SD strain, tests were carried out to examine acute toxicity following oral, subcutaneous or intraperitoneal administration of LEM-HT. The resulting $LD_{50}$ values were as follows:

|  | Male | Female |
|---|---|---|
| Oral administration: | 17.2 | 15.8 |
| Subcutaneous administration: | 4.5 | 5.2 |
| Intraperitoneal administration: | 3.3 | 3.1 |

(unit: g/kg)

Following oral and subcutaneous administration, both male and female rats died after one, and after two days, respectively, but differences were seen following intraperitoneal administration, in that males died in two days and females in three days. Administration of LEM-HT in a large dose caused a strong inflammation reaction at the administration site, irrespective of the mode of administration. More specifically, oral administration caused bleeding in the digestive tracts; subcutaneous administration resulted in necroses at the femoral regions; and intraperitoneal administration resulted in adhesion of the liver with the diaphragm. From such a pathology, it is assumed that an abrupt change resulting from the administration finally caused physical strength to fail, and resulted in death.

As a result of the acute toxicity tests as reported above, LEM-HT was confirmed to be as safe as a general food.

(9) Clinical Trial on AIDS patients - No 1

Using LEM-HT, clinical therapeutic trials were carried out on AIDS patients, although the number of patients in the trial were low. Tests were carried out at the following institution and by the following testers.

Testing institution:

Emperor's College of Traditional
Oriental Medicine (U.S.A)

Testers:

Dr. M. Moslek
Dr. Kevin Lance Jones

(i) Case 1

A case who had a serious fever and feeling of fatigue consulted a doctor. As a result of clinical tests, the case was recognized to have HTLV-III antibodies, and was thus diagnosed as having AIDS. Before administration of LEM-HT, the number of $T_4$ cells was 1,250 cells/mm$^3$. LEM-HT was orally administered in an mount of 6 g every day. As a result, the number of $T_4$ cells after 30 days was restored to 2,045 cells/mm$^3$; and the number of $T_4$ cells after 60 days was 2,542 cells/mm$^3$, resulting in relief of the subjective symptoms.

(ii) Case 2

A case of Karposi's sarcoma caused by infection with human immunodeficiency viruses. Before administration of LEM-HT, the number of $T_4$ cells was 822 cells/mm$^3$. LEM-HT was orally administered in an amount of 9 g every day. As a result, the number of $T_4$ cells increased up to 1,050 cells/mm$^3$ towards recovery, but the subject developed complications due to pneumonia and died.

(iii) Case 3

A case who had no subjective symptoms, but was found in clinical tests to be infected with HTLV-III. The case was in the state of what is called a carrier. LEM-HT was orally aministered in an amount of 6 g every day. As a result of administration for 60 days, virus antigens disappeared.

After the above clinical therapeutic trials, the doctors who had operated the examination reported that LEM-HT, which originates from 100% natural products, was safe, and resulted in no side effect to the subjects under investigation.

(10) Clinical Trial to AIDS patients - No 2

i) Testing institutions and testing doctors

| Testing institution | Testing doctor |
| --- | --- |
| Sangyo Medical College | Akira Shirahata |
| Tohoku University, School of Medicine | Kazuo Mori |
| Hama-no-machi Hospital | Kunio Kishida |
| Kagoshima University, School of Medicine | Ikuro Maruyama |

ii) Test Method

Among 18 cases suffering from hemophilia and to which LEM-HT had been administered at the above four institutions, 13 cases were tested (hemophilia A: 11 cases; hemophilia B: 2 cases), to whom LEM-HT had been administered for 3 months or more. The age of the cases to be tested ranged from 13 to 49. Nine (9) cases were grouped as AC (asymptomatic carrier), 1 case ARC (AIDS-related complex), 2 cases AIDS, and 1 case HIV antibody negative. The total daily dose of LEM-HT was 9 g, administered three times a day. Then the effect of LEM-HT on the immunological competence of these haemophiliac cases infected with HIV was examined.

iii) Results

(a) As to $CD_{4/8}$ ratio, an increase was observed in 3 cases among the 9 cases of AC, a decrease, in 2 cases, and a temporary increase, in 1 case among the remaining 4 cases.
(b) As to $CD_4$ absolute number, an increase was seen in 2 cases among the 9 cases of AC, a decrease, in 3 cases, and a temporary increase, in the remaining 4 cases. No change was seen in the 1 case of ARC, but a tendency of decrease, in the 2 cases of AIDS.
(c) As to lymphocyte blastogenesis reaction by PHA, Con A, or PWM, an increase was seen in 5 cases among 8 cases of AC, a decrease, in 1 case, and no change, in 2 cases. No change was also seen in the ARC cases, but a remarkable increase was seen in the 2 cases of AIDS, resulting in a relief of candidiasis from a clinical symptom.
(d) In the 1 case of HIV antibody negative, increases were seen after administration of LEM-HT, as to all the $CD_{4/8}$ ratio, $CD_4$ absolute number, and lymphocyte blastogenesis reaction.
(e) No side effect was seen which was considered to be caused by the present agent.
(f) Conclusion
LEM-HT had a remarkable influence particularly on the lymphocyte blastogenesis reaction. In some cases, an increase of $CD_4$ was also seen and a clinical improvement effect was obtained in all the cases of AIDS. Thus, the present agent is deemed to be an agent highly worth while to be tried as a prophylatic or therapeutic agent.

Example 2

(1) Fractionation of LEM-HT:

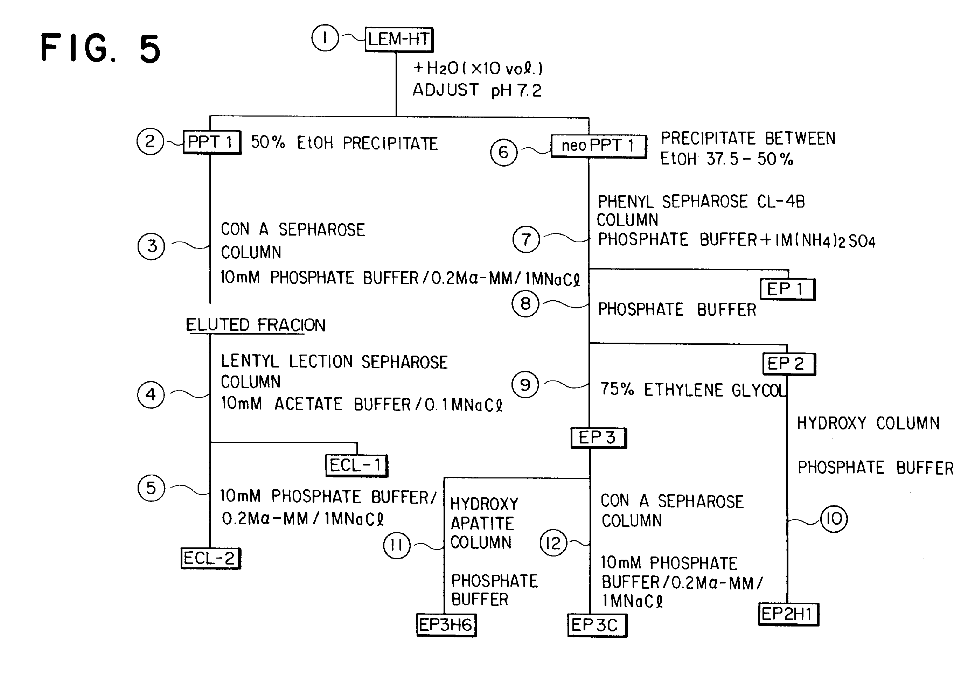

LEM-HT was prepared in the same manner as Example 1 described above. The resulting LEM-HT was fractionated according to the flow chart as shown in Fig. 5.
(i) A 10-fold volume of water was added to LEM-HT, and the resulting suspension was adjusted to pH 7.2.
(ii) To this suspension, ethyl alcohol was added to give an alcohol concentration of 50%, followed by centrifugal separation to obtain a precipitate, which was designated as PPT1.
(iii) This precipitate was passed through a Con A Sepharose column and adsorbed thereon, followed by elution using a phosphate buffer solution (pH 7.2) containing 1M NaCl and 0.2M α-methyl mannoside.

(iv) The resulting eluate was further passed through a lentyl lectin Sepharose column, and a fraction eluted using 10mM acetate buffer solution (pH 6.0) containing 0.1M sodium chloride was designated as ECL1.

(v) The adsorbed material was further eluted using a phosphate buffer solution containing 1M NaCl, 0.2M $\alpha$-methyl mannoside, and the resulting fraction was designated as ECL2.

(vi) A 10-fold volume of water was added in LEM-HT, and the resulting suspension was adjusted to pH 7.2. Thereafter, ethyl alcohol was added to give an alcohol concentration of 37.5 %, followed by centrifugal separation to remove a precipitate. In the resulting supernatant, ethyl alcohol was further added so as to give an alcohol concentration of 50%, followed by centrifugal separation to obtain a precipitate. A fraction soluble at an alcohol concentration of 37.5% and insoluble at 50% was collected, which was designated as neoPPT1.

(vii) The neoPPT1 was passed through a phenyl Sepharose CL-4B column, and a fraction eluted using a phosphate buffer solution (pH 6.8) was designated as EP1.

(viii) A fraction further eluted using a phosphate buffer solution (pH 6.8) was designated as EP2.

(ix) A fraction further eluted using 75% ethylene glycol was designated as EP3.

(x) The EP2 was adsorbed on a hydroxy apatite column, and a fraction eluted using a phosphate buffer solution (pH 6.8) was designated as EP2H1.

(xi) The EP3 was adsorbed on a hydroxy apatite column, and a fraction eluted using a phosphate buffer solution (pH 6.8) was designated as EP3H6.

(xii) The EP3 was adsorbed on a Con A Sepharose column, and a fraction eluted using a phosphate buffer solution containing 1M NaCl and 0.2M $\alpha$-methyl mannoside was designated as EP3C.

(2) Tests on inhibitory effect to infection with human immunodeficiency viruses by each fraction:

i) Test method

Once ATH8 cells are infected with HIV, viable cells gradually decrease. Then, in a culture medium of ATH8 cells ($2 \times 10^5$ per culture), the above fractions were each added so as to give a concentration of from 0 to 200 $\mu$g/m$\ell$. In the resulting cell mixture, HIV (2,000 virus particles per cell) was inoculated, and the decrease in viable cells was compared with that of controls which were not infected with HIV.

ii) Test results

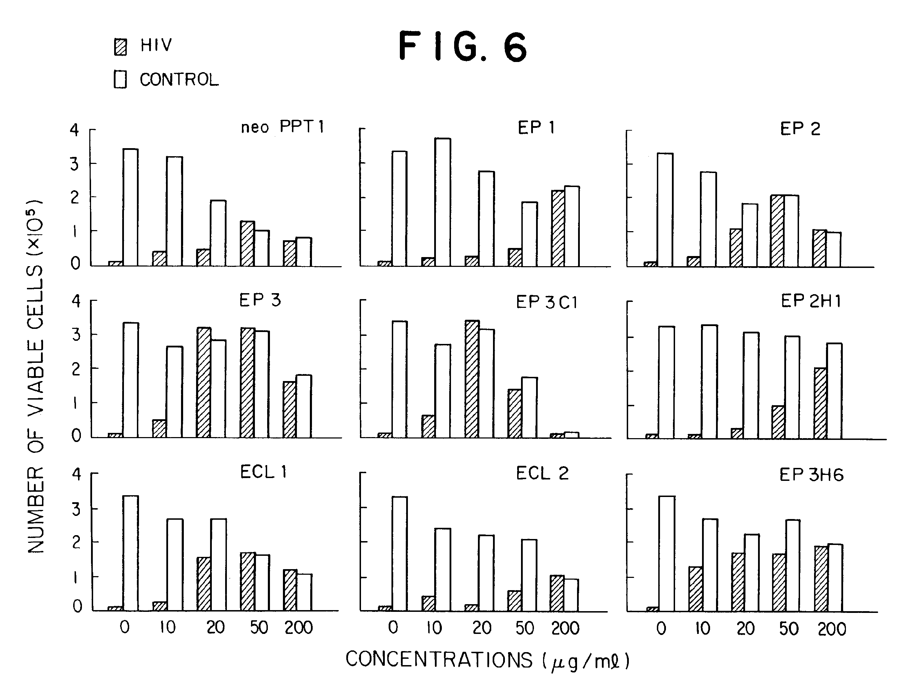

Test results obtained are shown in Fig. 6. In Fig. 6, the black bar represents a group brought into contact with HIV, and the white bar a group of controls. The ordinate therein indicates the number of viable cells ($\times 10^5$), and the abscissa the concentration of the specimens added ($\mu$g/m$\ell$).

As will be evident from Fig. 6, anti-HIV activity was seen in all the respective fractions of LEM-HT.

In particular, using EP3 fraction, it was possible to completely inhibit the decrease in viable cells which is due to the infection with HIV, when the fraction was in a concentration of 20 to 50 $\mu$g/m$\ell$.

A good anti-HIV effect was also seen in the fractions EP2, EP3C and EP3H6.

Thus, the respective fractions of LEM-HT showed a superior anti-HIV effect even in a low concentration of about 20 to 50 $\mu$g/m$\ell$.

(3) Tests to elucidate the operation and mechanism of the substance of the present invention:

i) Test method

To reverse transcriptase (RT) prepared from human immunodeficiency viruses, neoPPT1 and EP3 were each added so as to give various concentrations from 0 to 100 $\mu$g/m$\ell$, and the activities of the referse transcriptase at each concentration were measured.

ii) Test results

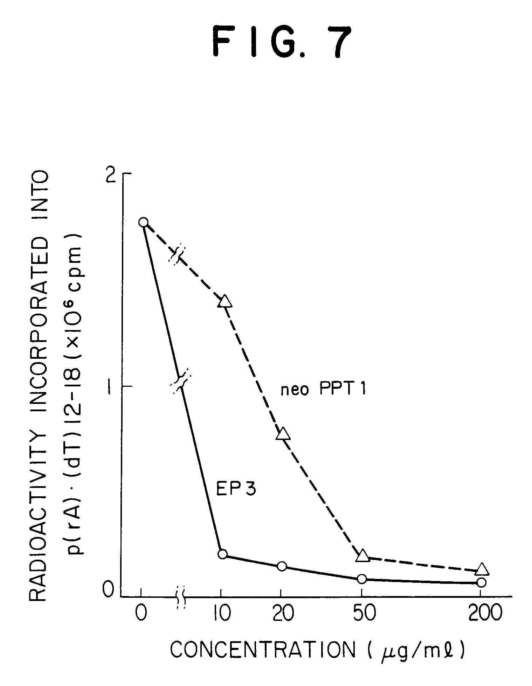

Test results obtained are shown in Fig. 7. In Fig. 7, the dotted line with triangles represents the results on neoPPT1, and the dotted line with circles the results on EP3. The ordinate in Fig. 7 also represents the amount of marker necleotide incorporated into DNA, and the abscissa the concentration of neoPPT1 or EP3.

As will be evident from the results, neoPPT1, when used in a low concentration, did not show much RT inhibitory activity. EP3, however, showed a good inhibitory activity even at 10 $\mu$g/m$\ell$. This indicates that EP3 inhibits the activity of the reverse transcriptase, which transcribes transferring the RNA genome of the viruses into DNA, even in a low concentration, and thus inhibits the multiplication of viruses.

(4) Analysis of EP3:

Elementary analysis and analysis of inorganic components were carried out on EP3.

i) Results of elementary analysis

The following was found as a result of the elementary analysis.
C: 44.97 %; H: 3.81 %; N: 1.88 %; ash content: 5.70 %; O: 43.64 %

ii) Results of analysis of inorganic components

The following was found as a result of analysis of the components according to an inductively coupled plasma method and atomic abasorption spectrophotometry.
P: 0.52 %; S: 0.34 %; Mg: 0.03 %; Ca: 0.21 %, K: 0.34 %; Na: 2.17%
Thus, EP3 was identified to be a substance composed of the elements P, S, Mg, Ca, K and Na, in addition to the organic matter.
As will be evident from the test results and clinical therapeutic trials as reported in the above, the present therapeutic agent was confirmed to have a sufficient effect on human immunodeficiency viruses. What should also be mentioned specially in the present invention is that, although conventional substances having an antiviral action have had so strong a toxicity and side effect that they often cannot in practice be used, the present therapeutic agent, which is an extract from 100 % natural products, has a very high safety. In addition, the present therapeutic agent has been confirmed to be effective by oral administration, and can inhibit the activity of the reverse transcriptase of human immunodeficiency viruses even in a low concentration, thereby suppressing the multiplication of human immunodeficiency viruses, so that it can promise a good prophylactic or therapeutic effect against AIDS.

## Claims

1. A therapeutic agent for the treatment of human immunodeficiency virus (HIV) infections comprising as an active component a hot-water-soluble extract of the mycelia of fungi of the genus <u>Basidiomycetes</u>, said active component being soluble at an aqueous ethanol concentration of 37.5 % and insoluble at an aqueous ethanol concentration of 50 %.

2. A therapeutic agent according to claim 1, which is an extract of the Basidiomycete fungus <u>Lentinus edodes</u>.

3. A therapeutic agent according to claim 1 or claim 2 wherein said active component comprises a mixture comprising sugar, protein and an inorganic component.

4. A therapeutic agent according to claim 3, wherein said inorganic component comprises P, S, Mg, Ca, K and Na.

5. A therapeutic agent according to any one of claims 1 to 4 wherein said active component has the following elementary composition:
C: 44.97,%, H: 3.81 %, N: 1.88 %, ash content: 5.70 % and 0: 43.64 %, and contains as said inorganic component P: 0.52 %, S: 0.34 %, Mg: 0.03%, Ca: 0.21 %, K: 0.34 % and Na: 2.17 %.

6. A process for the preparation of a therapeutic agent as claimed in any one of claims 1 to 5, said process comprising the steps of:
   (a) culturing mycelia of Basidiomycetes;
   (b) causing the said mycelia to undergo autolysis;
   (c) extracting the mycelial contents using hot water;
   (d) subjecting the resulting extract to alcohol precipitation; and
   (e) collecting a fraction which is soluble at an aqueous ethanol concentration of 37.5 % and insoluble at an aqueous ethanol concentration of 50 %.

7. A process according to claim 6, wherein said mycelia of Basidiomycetes are cultured using a medium containing true grasses.

8. A process according to claim 6 or claim 7, wherein said Basidiomycete fungus is Lentinus edodes.

9. A process according to any one of claims 6 to 8, comprising the further step of fractionating by column chromatography the ethanol precipitate of step (e) whereby to collect an active fraction.

10. A process according to claim 9, wherein said ethanol precipitate of step (e) is passed through a phenyl sepharose column to collect a fraction eluted using 75 % ethylene glycol.

11. Use for the preparation of a therapeutic agent for use in the treatment of human immunodeficiency virus (HIV) infections, of a hot-water-soluble extract of the mycelia of fungi of the genus Basidiomycetes said extract being soluble at an aqneous ethanol concentration of 37.5 % and insoluble at an aqueous ethanol concentration of 50 %.


**Patentansprüche**

1. Therapeutisches Mittel zur Behandlung von Infektionen mit humänem Immundefekt-Virus (HIV), welches als eine aktive Komponente einen heißwasserlöslichen Extrakt der Myzele von Pilzen der Klasse Basidiomycetes umfaßt, wobei besagte aktive Komponente bei einer Konzentration von wäßrigem Ethanol von 37,5% löslich und bei einer Konzentration von wäßrigem Ethanol von 50% unlöslich ist.

2. Therapeutisches Mittel nach Anspruch 1, dadurch gekennzeichnet, daß es ein Extrakt des Basidiomyceten-Pilzes Lentinus edodes ist.

3. Therapeutisches Mittel nach Anspruch 1 oder Anspruch 2, dadurch gekennzeichnet, daß besagte aktive Komponente ein Gemisch umfaßt, das Zucker, Protein und eine anorganische Komponente umfaßt.

4. Therapeutisches Mittel nach Anspruch 3, dadurch gekennzeichnet, daß besagte anorganische Komponente P, S, Mg, Ca, K und Na umfaßt.

5. Therapeutisches Mittel nach einem der Ansprüche 1 bis 4, dadurch gekennzeichnet, daß besagte aktive Komponente die folgende Elementarzusammensetzung besitzt:
C: 44,97 %, H: 3,81 %, N: 1,88 %, Aschegehalt: 5,70 % und O: 43,64 %, und als besagte anorganische Komponente P: 0,52 %, S: 0,34 %, Mg: 0,03 %, Ca: 0,21 %, K: 0,34 % und Na: 2,17 % enthält.

6. Verfahren zur Herstellung eines therapeutischen Mittels nach einem der Ansprüche 1 bis 5, dadurch gekennzeichnet, daß besagtes verfahren die Schritte umfaßt:
(a) Kultivieren von Myzelen von Basidiomycetes;
(b) Bewirken, daß besagte Myzele Autolyse durchlaufen;
(c) Extrahieren der Myzelinhalte unter verwendung von heißem Wasser;
(d) Unterwerfen des resultierenden Extraktes unter Alkoholausfällung; und
(e) Sammeln einer Fraktion, die bei einer Konzentration von wäßrigem Ethanol von 37,5% löslich und bei einer Konzentration von wäßrigem Ethanol von 50% unlöslich ist.

7. Verfahren nach Anspruch 6, dadurch gekennzeichnet, daß besagte Myzele von Basidiomycetes unter verwendung eines Mediums kultiviert werden, das echte Gräser enthält.

8. Verfahren nach Anspruch 6 oder Anspruch 7, dadurch gekennzeichnet, daß besagter Basidiomyceten-Pilz Lentinus edodes ist.

9. Verfahren nach einem der Ansprüche 6 bis 8, dadurch gekennzeichnet, daß es den weiteren Schritt der Fraktionierung des Ethanolniederschlages von Schritt (e) durch Säulenchromatographie umfaßt, um dadurch eine aktive Fraktion zu sammeln.

10. Verfahren nach Anspruch 9, dadurch gekennzeichnet, daß besagter Ethanolniederschlag von Schritt (e) durch eine Phenyl-Sepharose-Säule hindurchgegeben wird, um eine Fraktion zu sammeln, die unter Verwendung von 75% Ethylenglykol eluiert wird.

**11.** Verwendung zur Herstellung eines therapeutischen Mittels zur Verwendung bei der Behandlung von Infektionen mit humanem Immundefekt-Virus (HIV) eines heißwasserlöslichen Extraktes der Myzele von Pilzen der Klasse <u>Basidiomycetes</u>, wobei besagter Extrakt bei einer Konzentration von wäßrigem Ethanol von 37,5% löslich und bei einer Konzentration von wäßrigem Ethanol von 50% unlöslich ist.

**Revendications**

**1.** Agent thérapeutique pour le traitement des infections par le virus de l'immunodéficience humaine (HIV), comprenant comme composant actif un extrait soluble dans l'eau chaude des mycéliums de champignons du genre <u>Basidiomycetes</u>, ledit composant actif étant soluble dans de l'éthanol aqueux à la concentration de 37,5 % et insoluble dans de l'éthanol aqueux à la concentration de 50 %.

**2.** Agent thérapeutique selon la revendication 1 qui est un extrait du champignon basidiomycète <u>Lentinus edodes</u>.

**3.** Agent thérapeutique selon la revendication 1 ou 2, dans lequel ledit composant actif comprend un mélange comprenant un sucre, une protéine et un composant minéral.

**4.** Agent thérapeutique selon la revendication 3, dans lequel ledit composant minéral comprend P, S, Mg, Ca, K et Na.

**5.** Agent thérapeutique selon l'une quelconque des revendications 1 à 4, dans lequel ledit composant actif a la composition élémentaire suivant :
C : 44,97 %, H : 3,81 %, N : 1,88 %, teneur en cendres : 5,70 % et O : 43,64 %, et contient comme dit composant minéral P : 0,52 %, S : 0,34 %, Mg : 0,03 %, Ca : 0,21 %, K : 0,34 % et Na : 2,17 %.

**6.** Procédé pour la préparation d'un agent thérapeutique selon l'une quelconque des revendications 1 à 5, lequel procédé comprend les étapes consistant à :
(a) cultiver des mycéliums de Basidiomycètes ;
(b) provoquer l'autolyse dudit mycélium ;
(c) extraire le mycélium avec de l'eau chaude ;
(d) soumettre l'extrait obtenu à une précipitation pour l'alcool ; et
(e) recueillir une fraction qui est soluble dans de l'éthanol aqueux à la concentration de 37,5 % et insoluble dans de l'éthanol aqueux à la concentration de 50 %.

**7.** Procédé selon la revendication 6, dans lequel on cultive lesdits mycéliums de Basidiomycètes en utilisant un milieu contenant des graminées.

**8.** Procédé selon la revendication 6 ou 7, dans lequel ledit champignon Basidiomycète est <u>Lentinus edodes</u>.

**9.** Procédé selon l'une quelconque des revendications 6 à 8, comprenant l'étape additionnelle de fractionnement par chromatographie sur colonne du précipité éthanolique de l'étape (e) afin de recueillir une fraction active.

**10.** Procédé selon la revendication 9, dans lequel on fait passer ledit précipité éthanolique de l'étape (e) à travers une colonne de phénylsépharose pour recueillir une fraction éluée avec de l'éthylèneglycol à 75 %.

**11.** Utilisation pour la préparation d'un agent thérapeutique destiné au traitement des infections provoquées par le virus de l'immunodéficience humaine (HIV) d'un extrait soluble dans l'eau chaude des mycéliums de champignons du genre <u>Basidiomycetes</u>, ledit extrait étant soluble dans l'éthanol aqueux à la concentration de 37,5 % et insoluble dans l'éthanol aqueux à la concentration de 50 %.

# F I G. I

EP 0 370 673 B1

# F I G. 2

```
LEM-HT 10.0g
    | + 200 mℓ WATER
    | SPIN 6.000 rpm, 20min
    |_____ ppt
 sup                                                    | FREEZE DRY
    | + EtOH (FINAL EtOH CONC, 10%), 4°C,               | [E-O] (EtOH 0%)
    | 20 min SPIN 7.000 rpm, 20min
    |_____ ppt
 sup                                                    | FREEZE DRY
    | + EtOH (FINAL EtOH CONC, 20%)                     | [E-1] (EtOH 0-10%)
    | SPIN 7.000 rpm, 20 min
    |_____ ppt
 sup                                                    | FREEZE DRY
    | + EtOH (FINAL EtOH CONC, 30%)                     | [E-2] (EtOH 10-20%)
    | SPIN 7.000 rpm, 20min
    |_____ ppt
 sup                                                    | FREEZE DRY
    | + EtOH (FINAL EtOH CONC, 40%)                     | [E-3] (EtOH 20-30%)
    | SPIN 7.000 rpm, 20 min
    |_____ ppt
 sup                                                    | FREEZE DRY
    | + EtOH (FINAL EtOH CONC, 50%)                     | [E-4] (EtOH 30-40%)
    | SPIN 7.000 rpm, 20min
    |_____ ppt
 [sup]                                                  | FREEZE DRY
                                                        | [E-5] (EtOH 40-50%)
```

# FIG. 3

# FIG. 4

16

EP 0 370 673 B1

# FIG. 5

(1) — LEM-HT

+ H₂O ( ×10 vol. )
ADJUST pH 7.2

(2) — PPT 1   50% EtOH PRECIPITATE

(6) — neo PPT 1   PRECIPITATE BETWEEN
EtOH 37.5 – 50%

(3) — CON A SEPHAROSE
COLUMN

10mM PHOSPHATE BUFFER / 0.2Mα-MM / 1M NaCℓ

(7) — PHENYL SEPHAROSE CL-4B
COLUMN
PHOSPHATE BUFFER + 1M (NH4)2 SO4

EP 1

(8) — PHOSPHATE BUFFER

ELUTED FRACION

(4) — LENTYL LECTION SEPHAROSE
COLUMN
10mM ACETATE BUFFER / 0.1M NaCℓ

EP 2

(9) — 75% ETHYLENE GLYCOL

HYDROXY COLUMN

PHOSPHATE BUFFER

ECL-1

EP 3

(5) — 10mM PHOSPHATE BUFFER /
0.2Mα-MM / 1M NaCℓ

HYDROXY
APATITE
COLUMN

(11) —

PHOSPHATE
BUFFER

CON A SEPHAROSE
COLUMN

(12) —

10mM PHOSPHATE
BUFFER / 0.2Mα-MM /
1M NaCℓ

(10)

ECL-2

EP3H6

EP 3C

EP2H1

FIG. 6

# F I G. 7